# EUROPEAN PATENT SPECIFICATION

(11) **EP 0 579 434 B1**
(45) Date of publication and mention of the grant of the patent: **18.10.1995**
(21) Application number: 93305277.1
(22) Date of filing: 06.07.1993
(51) Int. Cl.: C07C 409/16

(54) **Dialkyl peroxide, cross-linking composition containing the same, method for cross-linking, and electric power cable**
Dialkylperoxide, Vernetzungsmittelzusammensetzungen, die sie enthalten, Verfahren zum Vernetzen und elektrisches Kabel
Dialkyl peroxides, composition de réticulation les contenant, procédé de réticulation et câble électrique

(30) Priority: 16.07.1992 JP 210728/92; 10.09.1992 JP 242277/92; 19.05.1993 JP 139261/93
(43) Date of publication of application: 19.01.1994
(73) Proprietor: NOF CORPORATION, Tokyo 100 (JP)
(72) Inventor: Suyama, Shuji, Chita-gun, Aichi-ken (JP); Ishigaki, Hideyo, Chita-gun, Aichi-ken (JP); Matsushima, Masaru, Chita-gun, Aichi-ken (JP); Naito, Satoshi, Chita-gun, Aichi-ken (JP); Ooto, Noriyoshi, Chita-gun, Aichi-ken (JP); Okada, Hiroshi, Tokoname-shi, Aichi-ken (JP)
(74) Representative: Smith, Norman Ian

(56) References cited:
- WO-A-91/07387
- DE-A- 4 010 893
- FR-A- 2 464 948

## Description

This invention relates to a dialkyl peroxide which is a novel compound possessing a double bond and a peroxide group. The dialkyl peroxide is useful as a cross-linking agent for an ethylene type polymer and as a polymerization initiator for the synthesis of a graft polymer from an ethylene type polymer. This invention further relates to a method for the production of a crosslinked ethylene type polymer by heating an ethylene type polymer in combination with the dialkyl peroxide mentioned above. This method allows a cross-linked ethylene type polymer to be produced with an improved cross-linking degree without scorching. This invention also relates to an electric power cable which is provided with an insulating layer of cross-linked ethylene type polymer produced by the method mentioned above.

As peroxides possessing a double bond, Japanese Patent Public Disclosure No. 52-153916 discloses t-alkylperoxy-2-alkyl allyl carbonate, Japanese Patent Public Disclosure No. 62-197407 discloses alkyl-t-alkylperoxy fumarate, Japanese Patent Public Disclosure No. 61-91209 discloses t-alkylperoxy methacroyloxy ethyl carbonate, and Japanese Patent Public Disclosure No. 62-1733 discloses t-alkyl-isopropenyl cumyl peroxide, for example.

These peroxides are used as a polymerization initiator for vinyl monomers, a polymerization initiator for the synthesis of graft polymers, and a cross-linking agent for ethylene type polymers.

As cross-linking agents for ethylene type polymers, such dialkyl type peroxides as, for example, dicumyl peroxide (DCP), di-t-butyl peroxide (DBP), t-butylcumyl peroxide (BCP), bis(α-t-butyl peroxy isopropyl)benzene (DIP), 2,5-bis(t-butylperoxy)-2,5-dimethyl hexane (25B), 2,5-bis(t-butylperoxy)-2,5-dimethylhexin-3 (25YB), and isopropylcumyl-t-butyl peroxide (IPC) are known to the art.

As cross-linking agents which have a low vapor pressure and assume a liquid state at normal room temperature, Japanese Patent Publication No. 2-31106 discloses an organic peroxide mixture consisting of 25 to 10 wt% of bis(α-t-butylperoxy isopropyl)benzene and 75 to 90 wt% of isopropyl cumyl-t-butyl peroxide, Japanese Patent Public Disclosure No. 54-132644 discloses an alkyl group-substituted dicumyl peroxide, and The Journal of Organic Chemistry, Vol. 38, No. 25, p. 4219 (1973) discloses t-butyl(1,1-dimethyl-1-hydroxyethyl)peroxide (DAPO) and 2-t-butylperoxy-2-methylpropyl acetate (BPACE), for example.

The so-called rubber-plastic electric power cables (hereinafter referred to simply as "electric power cables") are divided generally into those of the type provided on the periphery of a conductor with an inner semiconducting layer and an insulating layer and those of the type provided with an inner semiconducting layer, an insulating layer, and an outer semiconducting layer. These component layers are formed by causing a resinous composition formed by combining a polyethylene type resin with a cross-linking agent to be extruded in the form of a layer onto the periphery of a conductor by the use of an extruding device and subsequently heating the extruded layer thereby cross-linking the polyethylene type resin.

The aforementioned peroxides possessing a double bond have low thermal decomposition points and 10 hour half-life temperatures (the temperature T₁₀, at which one half of a given sample is decomposed in 10 hours) which are invariably lower than 120^{o}C. When a grafting reaction or cross-linking reaction is effected by the use of such a peroxide, therefore, the product of the reaction is not suitable use at a high reaction temperature, as shown below. When the peroxide disclosed in Japanese Patent Public Disclosure No. 61-91209 is copolymerized with a vinyl monomer and the resultant copolymer is melted and kneaded with a polyolefin to graft the polyolefin to the copolymer, raising the temperature to ensure thorough kneading leads to decomposition of the peroxide before the kneading is completed as desired. When the peroxide disclosed in Japanese Patent Public Disclosure No. 62-1733 is used for cross-linking a polyethylene, the crosslinking action proceeds (in the form of scorching) when the polyethylene and the peroxide are being kneaded.

When the conventional cross-linking agents referred to above are practically used as cross-linking agents, some problems arise as follows. DCP, BCP, DIP, 25B, and IPC are invariably liable to scorching.

DBP and BPACE are liable to volatilize while they are being combined and kneaded with an ethylene type polymer.

25YB, on being thermally decomposed, emits 2,5-dihydroxy-2,5-dimethylhexine-3, a solid compound which remains as an extraneous matter in the cross-linked product.

DCP and DIP are in a solid state at normal room temperature and, therefore, consume much time in such works as weighing and blending and, during the work of handling, allow easy inclusion therein of an extraneous matter and do not permit easy detection of the extraneous matter so included therein.

When the peroxides and DAPO which are disclosed in Japanese Patent Publication No. 2-31106 and Japanese Patent Public Disclosure No. 54-132644 are used as crosslinking agents for ethylene type polymers, the polymers in process of production are liable to undergo the phenomenon of scorching and the cross-linked products have low crosslinking degrees.

In the conventional electric power cable which is provided with an insulating layer and a semiconducting layer containing a cross-linked ethylene type polymer, the insulating layer and the semiconducting layer are liable to form an extraneous matter and undergo scorching during the production of the cable or the union of cable ends. The extraneous matter and the scorching both degrade the characteristics of the electric power cable. The voltages that electric power cables are required to withstand have become very high in recent years. For this reason, a high-density polyethylene having a high softening point is used for the insulating layer and the semiconducting layer of the electric power cable. In these circumstances, scorching poses a serious problem. Thus, it is desired to provide a cross-linking agent which does not induce scorching when it is mixed with a resin used for the formation of an insulating layer or a semiconducting layer, exhibits an outstanding cross-linking quality, and precludes the occurrence of an extraneous matter.

The present inventors, as a result of various studies for the solution of the drawbacks of the prior art described above, have synthesized a peroxide which is a novel compound having a high thermal decomposition point as compared with the conventional double bond-containing peroxides. They have confirmed that this peroxide overcomes the drawbacks mentioned above. The present invention has been perfected as a result.

To be specific, this invention relates to a dialkyl peroxide represented by the following formula: 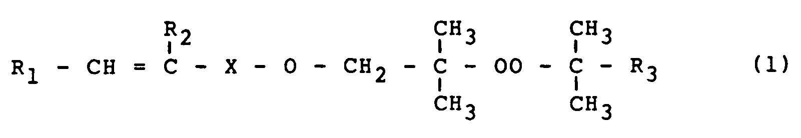
[wherein R₁ and R₂ independently stand for a hydrogen atom or a methyl group, R₃ stands for an alkyl group of 1 to 5 carbon atoms, and X stands for an alkylene group of 1 to 3 carbon atoms, -C(O)-, -CH₂-(O-CH₂-CHR₄)ₘ-OC(O)- {wherein R₄ stands for a hydrogen atom or a methyl group and m stands for an integer in the range of 0 to 10}, or -C(O)-(O-CH₂-CHR₅)ₙ-OC(O)- {wherein R₅ stands for a hydrogen atom or a methyl group and n stands for an integer in the range of 1 to 10}].

This invention also relates to a composition which has as essential components thereof a dialkyl peroxide represented by the formula (1) mentioned above and an ethylene type polymer.

This invention further relates to a method for cross-linking an ethylene type polymer by the use of a dialkyl peroxide represented by the formula (1) as a cross-linking agent.

This invention also relates to an electric power cable which is provided with an insulating layer obtained by cross-linking an ethylene type polymer by the use of a dialkyl peroxide represented by the formula (1) as a cross-linking agent.

The drawing attached hereto is a torque curve obtained using a curastometer in the experiments of Example 11 and Comparative Experiment 1 at 180^{o}C and 155^{o}C.

As concrete examples of the novel dialkyl peroxide according to this invention, there can be cited 2-t-butylperoxy-2-methylpropyl methacryloyloxy ethyl carbonate of the following formula: 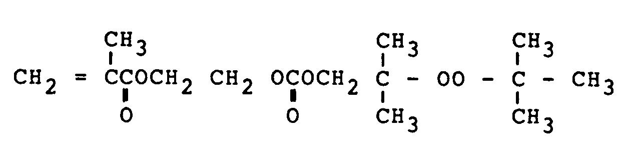
2-(1,1-dimethylpropylperoxy)-2-methylpropyl methacryloyloxy ethyl carbonate, 2-(1,1-dimethylbutylperoxy)-2-methylpropyl methacryloyloxy ethyl carbonate, 2-(1,1,3,3-tetramethylbutylperoxy)-2-methylpropyl methacryloyloxy ethyl carbonate, 2-t-butylperoxy-2-methylpropyl acryloyloxy ethyl carbonate, 2-t-butylperoxy-2-methylpropyl methacryloyloxy isopropyl carbonate of the following formula: 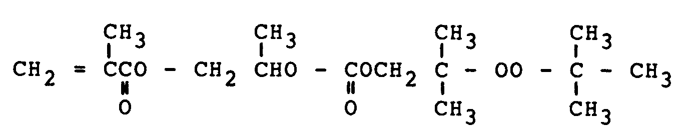
2-t-butylperoxy-2-methylpropyl methacryloylpoly(oxy isopropyl) carbonate of the following formula: 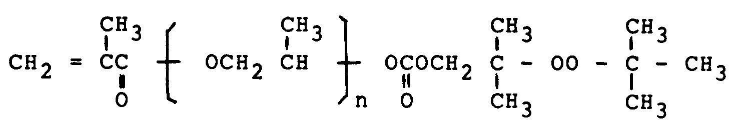
2-t-butylperoxy-2-methylpropyl methacryloyloxy ethoxy ethyl carbonate of the following formula: 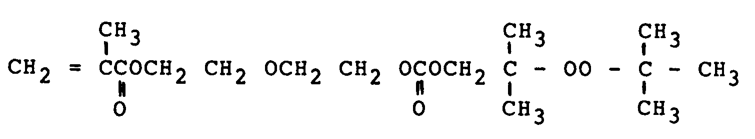
2-t-butylperoxy-2-methylpropyl methacrylate of the following formula: 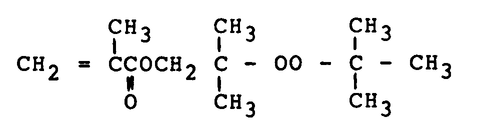
2-t-butylperoxy-2-methylpropyl acrylate, 2-t-butylperoxy-2-methylpropyl methallyl carbonate of the following formula: 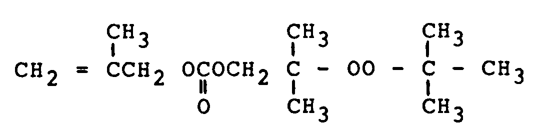
2-t-butylperoxy-2-methylpropyl allyl carbonate, 2-t-butylperoxy-2-methylpropyl crotonate, and 2-t-butylperoxy-2-methylpropyl allyl ether (hereinafter collectively referred to briefly as "dialkyl peroxides of this invention"). The dialkyl peroxides of this invention cited above are all viscous liquids at normal room temperature.

The dialkyl peroxide of this invention can be synthesized by the condensation reaction of t-butyl(1,1-dimethyl-2-hydroxy-ethyl) peroxide (DAPO) with chloroformate or an acid chloride, for example. Concrete examples of the chloroformate or acid chloride include 2-chloroformyl ethyl methacrylate, 2-chloroformyl ethoxy ethyl methacrylate, 2-chloroformyl poly(isopropoxy) isopropyl acrylate, 2-chloroformyl isopropyl methacrylate, methacryloyl chloride, acryloyl chloride, allyl chloroformate, and methallyl chloroformate.

The reaction conditions for the synthesis of the dialkyl peroxide of this invention will now be set out. The amount of chloroformate or acid chloride used with the DAPO is in the range of 0.8 to 1.2 mol based on 1 mol of DAPO. As a catalyst for the condensation, such a basic substance as pyridine or tertiary amine is used. This catalyst is used in an amount in the range of 1 to 2 mol based on 1 mol of the chloroformate or acid chloride. The reaction temperature is in the range of 10^{o}C to 40^{o}C, preferably 20^{o}C to 30^{o}C. The reaction time is in the range of 0.5 to 20 hours, preferably 1 to 8 hours. A reaction solvent may be used when necessary. The solvents which are effectively usable herein include paraffin type solvents and aromatic solvents, for example. The reactants, in process of reaction or after-treatment, may incorporate therein a polymerization inhibitor for the purpose of precluding the reactants from undergoing a polymerization reaction. The polymerization initiators which are effectively usable herein include 2,6-di-t-butyl-p-cresol and p-methoxyphenol, for example.

The chemical structure of the dialkyl peroxide of this invention can be identified from its infrared absorption spectrum or nuclear magnetic resonance spectrum. It can be analyzed for purity by liquid chromatography. The thermal decomposition temperature of this compound can be expressed as the 10-hour half-life temperature determined by a test for thermal decomposition.

When the dialkyl peroxide of this invention is copolymerized with a vinyl monomer copolymerizable therewith without inducing cleavage of the peroxide bond, a copolymer containing a peroxide group can be obtained. The copolymerizable vinyl monomers which are effectively usable herein include styrene and derivatives thereof, acrylic acid and acrylic esters, methacrylic acid and methacrylic esters, vinyl esters, halogenated vinyls, halogenated vinylidenes, conjugate diene, and vinyl ethers, for example. As concrete examples of these copolymerizable vinyl monomers, styrene, p-methylstyrene, o-chlorostyrene, p-methoxystyrene, acrylic acid, ethyl acrylate, n-butyl acrylate, methacrylic acid, methyl methacrylate, ethyl methacrylate, n-propyl methacrylate, n-butyl methacrylate, 2-ethylhexyl methacrylate, vinyl acetate, vinyl chloride, vinylidene chloride, vinylidene fluoride, butadiene, ethylene, tetrafluoroethylene, vinyl pyridine, and acrylamide may be cited.

The peroxide group-containing copolymer mentioned above may be further polymerized with another vinyl monomer to produce a graft copolymer.

A graft copolymer may be otherwise obtained by melting and kneading the peroxide group-containing copolymer mentioned above with a polyolefin.

Similarly to the conventional peroxide, the dialkyl peroxide of this invention may be used as a polymerization initiator or as a curing agent for unsaturated polyesters.

The ethylene type polymers which are effectively usable in the method for cross-linking contemplated by this invention include low-density polyethylene, high-density polyethylene, ethylene-propylene copolymer (EPR), ethylene-butene copolymer, ethylene-pentene copolymer, ethylene-vinyl acetate copolymer (EVA), ethylenepropylene-diene copolymer (EPDM), chlorinated polyethylene, ethylene-ethyl acrylate copolymer (EEA), ethylene-methyl methacrylate copolymer, ethylene-glycidyl methacrylate copolymer (EGMA), and ethylene-acrylonitrile copolymer, for example.

The amount of the dialkyl peroxide of this invention to be used in the method for cross-linking an ethylene type polymer as contemplated by this invention is in the range of 0.5 to 5 parts by weight, preferably 1 to 4 parts by weight, based on 100 parts by weight of the ethylene type polymer. The cross-linking degree is not sufficient if this amount is less than 0.5 part by weight. The cross-linking degree is so large as to embrittle the cross-linked product if the amount exceeds 5 parts by weight.

In the method for cross-linking as contemplated by this invention, the dially peroxide of this invention may be used in combination with any of the conventional peroxides popularly used as cross-linking agent. The conventional peroxides which are effectively usable for this purpose include dicumyl peroxide, t-butyl cumyl peroxide, 2,5-bis(t-butylperoxy)2,5-dimethyl hexane, 2,5-bis(t-butylperoxy)2,5-dimethylhexine-3, di-t-butyl peroxide, isopropyl cumyl-t-butyl peroxide, bis(α-t-butylperoxy isopropyl) benzene, and 3,3,6,6,9,9-hexamethyl-1,2,4,5-tetraoxy cyclononane.

The method for cross-linking contemplated by this invention allows additional use of a cross-linking auxiliary. The cross-linking auxiliaries which are effectively usable herein include such compounds as 2,4-diphenyl-4-methyl-1-pentene, terpinolene, ethylene glycol dimethacrylate, butylene glycol dimethacrylate, 1,3-butylene glycol dimethacrylate, trimethylol propane trimethacrylate, triethylene glycol dimethacrylate, tetraethylene glycol dimethacrylate, polyethylene glycol dimethacrylate, pentaerythritol trimethacrylate, ethylene glycol diacrylate, butylene glycol diacrylate, 1,3-butylene glycol diacrylate, trimethylol propane triacrylate, triethylene glycol diacrylate, tetraethylene glycol diacrylate, polyethylene glycol diacrylate, pentaerithritol triacrylate, triallyl cyanurate, triallyl isocyanurate, N,N'-m-phenylene bis-maleimide, and polybutadiene.

In the method for cross-linking according with the present invention, the reactants may incorporate therein various additives generally used in a crosslinking reaction such as, for example, antioxidant, pigment, stabilizer against the action of ultraviolet light, filler, plasticizer, and slip additive. The antioxidants which are effectively usable herein include phenol type compounds such as 4,4'-thio-bis(3-methyl-6-t-butyl phenol), 2,5-di-t-butyl hydroquinone, and 2,6-di-t-butyl-p-cresol, phosphorus type compounds, and sulfur type compounds such as bis(2-methyl-4-(3-n-alkyl thiopropionyloxy)-5-t-butyl phenyl) sulfide, 2,2'-thiodiethylene-bis-[3-(3,5-di-t-butyl-4-hydroxy phenyl)propionate], dilauryl thio-propionate, and distearyl thiopropionate. The amount of such an antioxidant to be incorporated is generally in the range of 0.05 to 1.0 part by weight, based on 100 parts by weight of the ethylene type polymer being cross-linked.

In the method for cross-linking according with the present invention, the temperature at which the crosslinking operation is carried out is generally in the range of 170^{o}C to 250^{o}C, preferably 180^{o}C to 220^{o}C.

The electric power cable of this invention comprises at least two resin layers provided on the periphery of a conductor and at least one of the resin layers is made of a resin obtained by cross-linking an ethylene type polymer by the use of a cross-linking agent of this invention.

The dialkyl peroxide of this invention is a high-temperature reaction type dialkyl peroxide which has a 10-hour half-life temperature in the range of 120^{o}C to 130^{o}C and a high thermal decomposition temperature as compared with the conventional peroxide possessing a double bond. It therefore can be used at high temperatures in the grafting reaction and the crosslinking reaction. In view of its properties described above, the dialkyl peroxide of this invention is useful as a polymerization initiator for vinyl monomers, as a raw material for graft polymerization, as a degrading agent for polyolefins, and as a cross-linking agent for ethylene type polymers.

The method for cross-linking according with the present invention enjoys high cross-linking efficiency and is unlikely to entail scorching. The dialkyl peroxide of this invention assumes a liquid state and exhibits low volatility at normal room temperature. When it is used as a cross-linking agent for an ethylene type polymer, therefore, the mixture prepared for the cross-linking operation is easy to handle during weighing, charging in the vessel and the like. Further, the mixture is unlikely to experience intrusion of extraneous matter, to degrade the working environment by volatilization, or to impair the uniform quality of the cross-linked product.

The electric power cable provided on the periphery of a conductor thereof with a resin layer produced by the use of the dialkyl peroxide of this invention as a cross-linking agent enjoys ideal electrical characteristics (AC breakdown strength) because the resin layer is free of scorching and because the resin forming the resin layer has a high cross-linking degree and forms no decomposition product harmful to the electrical characteristics. Since the resin layer has a high crosslinking degree, the amount of the cross-linking agent of this invention required to be used for obtaining a fixed cross-linking degree is small as compared with any of the conventional cross-linking agents. As a result, the amount of a gas emanating from the decomposition product of the cross-linking agent is proportionately small and the water tree can be decreased.

This invention will now be described more specifically below with reference to working examples. The abbreviations used in the following examples denote the following compounds.
- BPEMA:: 2-t-Butylperoxy-2-methylpropyl methacryloyloxy ethyl carbonate
- HPEMA:: 2-(1,1-Dimethyl butylperoxy)-2-methylpropyl methacryloyloxy ethyl carbonate
- BPEEMA:: 2-t-Butylperoxy-2-methylpropyl methacryloyloxy ethoxy ethyl carbonate
- BPPMA:: 2-t-Butylperoxy-2-methylpropyl methacryloyloxy isopropyl carbonate
- BPPPMA:: 2-t-Butylperoxy-2-methylpropyl methacryloyl penta(oxyisopropyl) carbonate
- BPMA:: 2-t-Butylperoxy-2-methylpropyl methacrylate
- BPA:: 2-t-Butylperoxy-2-methylpropyl acrylate
- BPAC:: 2-t-Butylperoxy-2-methylpropyl allyl carbonate
- BPMAC:: 2-t-Butylperoxy-2-methylpropyl methallyl carbonate
- BPC:: 2-t-Butylperoxy-2-methylpropyl crotonate
- DAPO:: t-Butyl(1,1-dimethyl-2-hydroxyethyl) peroxide (purity 98%)
- CEMA:: 2-Chloroformyl ethyl methacrylate (purity 92%)
- CEEMA:: 2-Chloroformyl ethoxyethyl methacrylate (purity 92%)
- CPMA:: 2-Chloroformyl isopropyl methacrylate (purity 92%)
- CPPMA:: 2-Chloroformyl tetraisopropoxy isopropyl methacrylate (purity 90%)
- CMA:: Methacryl chloride (purity 90%)
- CA:: Acryl chloride (purity 90%)
- CFA:: Allyl chloroformate (purity 98%)
- CFMA:: Methallyl chloroformate (purity 96%)
- CFC:: Crotonyl chloride (purity 92%)
- DCP:: Dicumyl peroxide (purity 99%)
- IPC:: Isopropylcumyl-t-butyl peroxide (m/p = 60/40, purity 95%)
- IPEC:: t-Butyl-m-isopropenylcumyl peroxide (purity 95%)
- DBP:: Di-t-butyl peroxide (purity 99%)
- BPACE:: 2-t-Butylperoxy-2-methylpropyl acetate
- BPMC:: t-Butylperoxy methacroyloxy ethyl carbonate (purity 91%)
- MSD:: 2,4-Diphenyl-4-methyl-1-pentene (purity 95%)
- TAIC:: Trially isocyanurate
- TBP:: 4,4'-Thio-bis(3-methyl-6-t-butyl phenol)

### EXAMPLE 1 (Sythesis of BPEMA)

In a four-neck flask having an inner volume of 500 ml and provided with a stirrer and a thermometer, 29.9 g (0.18 mol) of DAPO, 19.7 g (0.25 mol) of pyridine, and 31 g of hexane were kept stirred, a mixed solution consisting of 45.9 g (0.22 mol) of CEMA and 34 g of hexane was added dropwise at 7^{o}C to 14^{o}C, and the reactants were left reacting at 30^{o}C for seven hours. After the reaction, the reaction product was made to separate an organic phase by addition of 200 ml of an aqueous 5% hydrochloric acid solution. The organic phase was wahsed with an aqueous 0.5% hydrochloric acid solution, with an aqueous 10% potassium nitrate solution, and with water, and then dehydrated with anhydrous magnesium sulfate to produce 124 g of a transparent liquid. This liquid, on analysis by liquid chromatography (LC), was found to contain BPEMA in a purity of 47%, indicating a yield of 99% based on DAPO. The liquid was distilled under a vacuum to expel the solvent and then separated by LC to isolate the main component. By analysis of the infrared absorption spectrum (IR) and the nuclear magnetic resonance spectrum (NMR), this main component was identified to be BEEMA. This peroxide was dissolved in cumene in a concentration of 0.05 mol/liter and the solution was subjected to a thermal decomposition test. As a result, the T₁₀ of the sample was found to be 127^{o}C. The reaction conditions and the results of the test are shown in Table 1.
- IR:: 1750 cm⁻¹ (C = O bond), 1725 cm⁻¹ (C = O bond), 1640 cm⁻¹ (C = C bond), and 880 cm⁻¹ (O - O bond)
- NMR (δ, ppm) :: 1.20 [s, 9H], 1.26 [s, 6H], 1.95 [s, 3H], 4.17 [s, 2H], 4.3 ∼ 4.4 [m, 4H], 5.59 [s, 1H], and 6.14 [s, 1H]

### EXAMPLE 2 (Synthesis of HPEMA)

When the procedure of Example 1 was repeated, except that 34.3 g of 1,1-dimethylbutyl(1,1-dimethyl-2-hydroxyethyl) peroxide (purity 98%) was used in the place of DAPO, there was obtained 115 g of a solution in hexane of HPEMA in a purity of 48% (yield 90%). By analyzing the hexane solution in the same manner as in Example 1, the reaction product was identified to be HPEMA. As a result of a thermal decomposition test performed in the same manner as in Example 1, the T₁₀ of the product was found to be 122^{o}C. The reaction conditions and the results of the test are shown in Table 1.
- IR:: 1750 cm⁻¹ (C = O bond), 1725 cm⁻¹ (C = O bond), 1640 cm⁻¹ (C = C bond), and 880 cm⁻¹ (O - O bond)
- NMR (δ, ppm):: 0.92 [t, 3H], 1.4 ∼ 1.6 [m, 4H], 1.19 [s, 6H], 1.21 [s, 6H], 1.95 [s, 3H], 4.17 [s, 2H], 4.3 ∼ 4.4 [m, 4H], 5.59 [s, 1H], and 6.14 [s, 1H]

### EXAMPLES 3 AND 4 (Synthesis of BPEEMA and BPPMA)

The procedure of Example 1 was repeated, except that CEEMA (Example 3) and CPMA (Example 4) were each used in an amount of 0.22 mol in the place of CEMA. The results are shown in Table 1. The produced peroxides were identified by IR and NMR to be BPEEMA and BPPMA. The reaction conditions and the results of the test are shown in Table 1.

### EXAMPLE 5 (Synthesis of BPPPMA)

In a four-neck flask having an inner volume of 300 ml and provided with a stirrer and a thermometer, 10.0 g (0.06 mol) of DAPO, 6.6 g (0.08 mol) of pyridine, and 15 g of benzene were placed and then kept stirred, a mixed solution consisting of 34.1 g (0.07 mol) of CPPMA and 15 g of benzene was added dropwise thereto at 9^{o}C to 13^{o}C, and the reactants were left reacting at 30^{o}C for 8 hours. After the reaction, the reaction product was made to separate an organic phase by addition of 70 ml of an aqueous 2% hydrochloric acid solution. The organic phase was washed with an aqueous 2% hydrochloric acid solution, with an aqueous 10% potassium carbonate solution, and with water, and then dehydrated with anhydrous magnesium sulfate to produce 58 g of a transparent liquid. The purity was 50% and the yield 86%. By analysis performed in the same manner as in Example 1, the product was identified to be BPPPMA. The T₁₀ of the sample was found to be 127^{o}C. The reaction conditions and the results of the test are shown in Table 1.

### EXAMPLE 6 (Synthesis of BPMA)

In a four-neck flask having an inner volume of 100 ml and provided with a stirrer and a thermometer, 8.3 g (0.05 mol) of DAPO, 5.1 g (0.064 mol) of pyridine, and 5 g of hexane were placed and kept stirred, a mixed solution consisting of 7.0 g (0.06 mol) of CMA (purity 90%) and 5 g of hexane was added dropwise at 10^{o}C to 14^{o}C, and the reactants were left reacting at 20^{o}C for 0.5 hour. After the reaction, the reaction product was made to separate an organic phase by addition of 30 ml of an aqueous 5% hydrochloric acid solution. The organic phase was washed with an aqueous 10% potassium carbonate solution and with water and then dehydrated with anhydrous magnesium sulfate. The washed organic phase was concentrated under a vacuum to produce 60 g of a transparent liquid. The purity was 90% and the yield 47%. By analysis performed in the same manner as in Example 1, the product was identified to be BPMA. The T₁₀ of the sample was found to be 127^{o}C. The reaction conditions and the results of the test are shown in Table 1.

### EXAMPLES 7 TO 10 (Synthesis of BPA, BPAC, BPMAC, and BPC)

The procedure of Example 6 was repeated, except that CA (Example 7), CFA (Example 8), CFMA (Example 9), and CFC (Example 10) were each used in an amount of 0.06 mol in the place of CMA and the reaction temperature and the reaction time were changed. The reaction conditions and the results of the test are shown in Table 1. The produced peroxides were identified by IR and NMR to be those compounds shown in Table 1.

**Table 1**

| Example No. | Acid chloride or chromoformate | Formed peroxide | Temperature (^{o}C) | Time (h) | Yield (%) | T₁₀ (^{o}C) |
|---|---|---|---|---|---|---|
| 1 | CEMA | BPEMA | 30 | 7 | 99 | 127 |
| 2 | CEMA | HPEMA | 30 | 7 | 90 | 122 |
| 3 | CEEMA | BPEEMA | 30 | 7 | 95 | 127 |
| 4 | CPMA | BPPMA | 30 | 7 | 95 | 127 |
| 5 | CPPMA | BPPPMA | 30 | 8 | 86 | 124 |
| 6 | CMA | BPMA | 20 | 0.5 | 47 | 127 |
| 7 | CA | BPA | 20 | 0.5 | 49 | 127 |
| 8 | CFA | BPAC | 20 | 5 | 85 | 127 |
| 9 | CFMA | BPMAC | 20 | 5 | 88 | 127 |
| 10 | CFC | BPC | 30 | 7 | 93 | 127 |

### REFERENTIAL EXAMPLE 1 (Copolymerization of styrene by use of BPEMA)

A glass ampoule having an inner volume of about 20 ml was charged with 10.15 g of a styrene solution containing benzoyl peroxide in a concentration of 0.02 mol/liter and 0.51 g of a hexane solution of BPEMA (purity 39%) and, with the entrapped air displaced with nitrogen, was then sealed by fusion. The ampoule was kept immersed in a constant temperature bath kept at 80^{o}C for 9 hours. Then, the ampoule was removed from the bath, cooled, and opened by breaking the seal. Part of the reaction product was dissolved in benzene and the resultant solution was added dropwise to methanol to induce precipitation. The white solid precipitate consequently formed was separated by filtration and dried under a vacuum. When this white solid substance was analyzed by GPC, it was found to have a number average molecular weight of 95,000. When NMR (¹H-NMR) analysis was conducted on the white solid substance, a peak originating in the methyl hydrogen of a t-butyl group possessed by the peroxy part of BPEMA was observed at 1.2 ppm in addition to a peak due to a styrene structure unit in the spectrum. In the IR analysis of the white solid substance, an absorption by a carbonyl group was observed at 1740 cm⁻¹ in the spectrum. On the basis of these data, the white solid substance was identified to be a styrene copolymer incorporating therein the aforementioned peroxy part of BPEMA.

### REFERENTIAL EXAMPLE 2 (Grafting reaction of styrene to low-density polyethylene by use of BEEMA)

In a stainless steel reactor having an inner volume of 3 liters and provided with a thermometer, a Dimroth condenser, and a stirrer, 1,350 g of water was placed and 75 g of an aqueous 1% polyvinyl alcohol ("Gosenol KH-17" produced by Nippon Synthetic Chemical Industry Co., Ltd.) solution and 75 g of an aqueous 10% hydroxy apatite ("Supertight ST-10" produced by Nippon Chemical Industry Co., Ltd.) solution were dissolved in the water as suspending agents. In the resultant solution, 350 g of a low-density ethylene polymer having a particle diameter of 3 to 4 mm ("Sumikasen G401" produced by Sumitomo Chemical Co., Ltd.) and 135 g of styrene were placed and dispersed by stirring. They were kept immersed in the solution at 90^{o}C for two hours under a current of nitrogen. Separately, 1.0 g of a water-diluted benzoyl peroxide (purity 75%) ("Naiber BW" produced by Nippon Oil & Fats Co., Ltd.) and 12.4 g of a n-hexane-diluted BPEMA (purity 47%) were dissolved in 15 g of styrene. This solution was added to the mixture in the reactor at 80^{o}C and stirred therein. The resultant reaction mixture was heated at 80^{o}C for three hours. It was then further heated to 90^{o}C and kept at this temperature for one hour to complete the reaction in process. The reaction product was washed with water and dried to produce 486 g of a graft precursor. This graft precursor was kneaded at 220^{o}C for 10 minutes by the use of a labo plastomill (B-75 type mixer) to produce a graft polymer. The grafting efficiency of this graft polymer with respect to polystyrene was 48%.

### EXAMPLE 11 (Cross-linking of polyethylene)

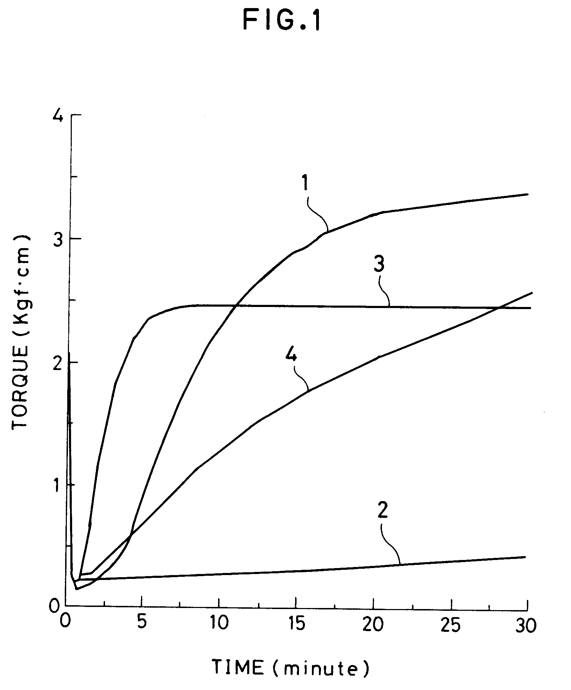

A composition was produced by mixing 500 g of a low-density polyethylene ("NUC-9025" produced by Nippon Unicar Co., Ltd.) with 14.7 g of BPEMA as a cross-linking agent. This composition was kneaded by means of a hot roll at about 110^{o}C for about 20 minutes. The treated composition was subjected to a heating test with an instrument ("JSR Curastometer, III model" produced by Toyo Baldwin K.K.). The maximum torque was measured at 180^{o}C. Separately, a sample of the treated composition was tested for scorching time at 155^{o}C. The scorching time was defined as the length of time required for the torque of a sample to rise from the minimum value to 10% of the maximum value at 180^{o}C. The results are shown in Table 2. The torque curves 1 and 2 obtained respectively at 180^{o}C and 155^{o}C are shown in Figure 1.

### EXAMPLES 12 TO 19 AND COMPARATIVE EXPERIMENTS 1 TO 6

The procedure of Example 11 was repeated, except that different cross-linking agents shown in Table 2 were used in the place of BPEMA. The results are shown in Table 2. The torque curves 3 and 4 obtained respectively at 180^{o}C and 155^{o}C in Comparative Experiment 1 are shown in Figure 1.

It is found by comparing the data of Examples 11 to 19 with the data of Comparative Experiments 1 to 6 that the cross-linked polyethylenes obtained by the method for cross-linking according with this invention possessed high cross-linking degrees and did not easily experience scorching.

### EXAMPLES 20 TO 22 AND COMPARATIVE EXPERIMENT 7

The procedure of Example 11 was repeated, except that different cross-linking agents shown in Table 2 were used in the place of BPEMA and different additives shown in Table 2 were used. The results are shown in Table 2.

It is found by comparing the data of Example 20 with the data of Comparative Experiment 7 that though both used an antioxidant, the cross-linked polyethylene produced by the method for cross-linking according with this invention had a high cross-linking degree and did not easily experience scorching. From the data of Examples 21 and 22, it is noted that combined use of the peroxide of this invention with conventional cross-linking auxiliaries in the cross-linking of ethylene enabled the produced cross-linked polyethylenes to enjoy decreases in the occurrence of scorching and increases in the cross-linking degree.

**Table 2**

| Example No. or Comparative Experiments No. | Cross-linking agent (g) | Additive (g) | Scorching time at 155^{o}C (min) | Maximum torque (kgf.cm) |
|---|---|---|---|---|
| Example 11 | BPEM 14.7 | - | 40 | 3.40 |
| Comparative Experiment 1 | DCP 12.5 | - | 3.5 | 2.48 |
| Comparative Experiment 2 | IPC 12.0 | - | 8.5 | 2.77 |
| Comparative Experiment 3 | IPEC 12.0 | - | 9.0 | 2.80 |
| Comparative Experiment 4 | BPACE 9.3 | - | 12.0 | 2.81 |
| Comparative Experiment 5 | DAPO 7.5 | - | 3.2 | 2.30 |
| Comparative Experiment 6 | BPMC 11.4 | - | 2.0 | 2.31 |
| Example 12 | BPEEMA 16.7 | - | 38 | 3.00 |
| Example 13 | BPPMA 15.4 | - | 38 | 3.02 |
| Example 14 | BPPPMA 26.1 | - | 39 | 2.88 |
| Example 15 | BPMA 10.7 | - | 39 | 3.35 |
| Example 16 | BPA 10.0 | - | 37 | 3.33 |
| Example 17 | BPAC 11.4 | - | 30 | 3.02 |
| Example 18 | BPMAC 12.0 | - | 34 | 3.11 |
| Example 19 | BPC 14.7 | - | 30 | 3.05 |
| Example 20 | BPEMA 14.7 | TBP 1.0 | 46 | 3.11 |
| Comparative Experiment 7 | DCP 12.5 | TBP 1.0 | 5.5 | 2.25 |
| Example 21 | BPEMA 14.7 | MSD 3.0 | 52 | 3.97 |
| Example 22 | HPEMA 14.7 | TAIC 3.0 | 23 | 4.22 |

### EXAMPLE 23

A composition was produced by mixing 500 g of a high-density polyethylene ("HDF 6080V" produced by Ace Polymer K.K.) with 8.8 g of BPEMA, 2 g of bis[2-methyl-4-(3-n-alkylthiopropionyloxy)-5-butyl phenyl] sulfide, and 3 g of N,N'-m-phenylene bis-maleimide. This composition was kneaded by means of a hot roll at about 130^{o}C for about 20 minutes. The kneaded composition was then treated in the same manner as in Example 11. The maximum torque of the composition at 180^{o}C was 5.63 kgf.cm. The scorching time of the composition at 155^{o}C was 37 minutes.

### COMPARATIVE EXPERIMENT 8

The procedure of Example 23 was repeated, except that 7.5 g of DCP was used in the place of BPEMA. The maximum torque of the composition at 180^{o}C was 3.75 kgf.cm and the scorching time of the composition at 155^{o}C was 3.2 minutes.

### EXAMPLE 24

A composition was produced by mixing 500 g of an ethylene-vinyl acetate copolymer ("Ultracene 625" produced by Toyo Soda K.K.) with 19.1 g of BPEMA and 1 g of TBP. This composition was kneaded by means of a hot roll at about 100^{o}C for about 20 minutes. It was then treated in the same manner as in Example 11. The maximum torque of this composition at 180^{o}C was 1.59 kgf.cm. The scorching time of this composition at 155^{o}C was 46 minutes.

### COMPARATIVE EXPERIMENT 9

The procedure of Example 24 was repeated, except that 15.0 g of IPC was used in the place of BPEMA. The maximum torque of the produced composition at 180^{o}C was 3.75 kgf.cm. The scorching time of the composition at 155^{o}C was 4.5 minutes.

It is found by comparing the data of Examples 23 and 24 with the data of Comparative Experiments 8 and 9 that similarly in the cross-linking of a high-density polyethylene and an ethylene-vinyl acetate copolymer, the compositions according with this invention produced high cross-linking degrees and did not easily experience scorching.

### EXAMPLE 25 (Production of electric power cable)

An electric power cable provided with crosslinked polyethylene insulated layers, rated at 6 kV and having a cross-sectional area of 38 mm² was produced by coating a conductor sequentially with an inner semiconducting layer of a composition of polyethylenevinyl acetate, acetylene black, and BPEMA (100/60/1 by weight ratio) formed by means of an extruding device, an insulating layer of a composition of low-density polyethylene, BPEMA, and TBP (100/3/0.2 by weight ratio) formed by extrusion at 145^{o}C by means of the extruding device, and an outer semiconducting layer of the same composition as that of the inner semiconducting layer formed similarly and then heating the superposed layers at 200^{o}C for 10 minutes thereby cross-linking the compositions of the layers. The AC breakdown voltage of the produced electric power cable was 270 kV. When a sample cut from the cross-linked insulating part of the cable was observed under a microscope, no "scorch" was detected. When the sample was left standing in xylene at 130^{o}C for five hours to extract a gel, the gel proportion was found to be 93%.

### COMPARATIVE EXPERIMENT 10

An electric power cable was produced by following the procedure of Example 25, except that a composition of low-density polyethylene, DCP, and TBP (100/2.5/0.2 by weight ratio) was used for the insulating layer in the place of the composition of low-density polyethylene, BPEMA, and TBP and this cable was tested in the same manner as in Example 25. The AC breakdown voltage of the produced electric power cable was 200 kV. When a sample cut from the cross-linked insulating part of the produced electric power cable was observed under a microscope, "scorch" was detected. When the sample was left standing in xylene at 130^{o}C for five hours to extract a gel, the gel proportion was found to be 88%.

It is found by comparing the data of Example 25 with the data of Comparative Experiment 10 that the electric power cable according with this invention experienced no scorch in the resin layers, showed high cross-linking degrees in the resin (insulation) layers, and manifested enhanced electrical characteristics as compared with the conventional electric power cable.

### EXAMPLES 26 TO 35 AND COMPARATIVE EXPERIMENTS 11 AND 12

Compositions for cross-linking were produced by mixing 1,000 g of a low density polyethylene (NUC-9025) with different amounts of a cross-linking agent. Each composition was placed in a polyethylene bag 0.05 mm in wall thickness, sealed tightly therein, and left standing at a room temperature of about 15^{o}C for 30 days. The amount of the composition that volatilized during the standing was determined by measuring the change in weight of the composition before and after the standing. The amounts of the cross-linking agent used in the compositions and the amounts of the compositions lost by volatilization are shown in Table 3. (The amount of volatilization was expressed by the ratio relative to the organic peroxide added.)

From the results shown in Table 3, it is noted that the composition according with this invention possessed lower volatility than di-t-butyl peroxide having substantially the same thermal decomposition temperature.

**Table 3**

| Example No. or Comparative Experiment No. | Cross-linking agent (g) | Amount of volatilization (%) |
|---|---|---|
| Example 26 | BPEMA 20 | 1 |
| Example 27 | HPEMA 20 | 0 |
| Example 28 | BPEEMA 20 | 0 |
| Example 29 | BPPMA 20 | 0 |
| Example 30 | BPPPMA 20 | 0 |
| Example 31 | BPMA 20 | 11 |
| Example 32 | BPA 20 | 19 |
| Example 33 | BPAC 20 | 17 |
| Example 34 | BPMAC 20 | 12 |
| Example 35 | BPC 20 | 10 |
| Comparative Experiment 11 | DBP 20 | 48 |
| Comparative Experiment 12 | BPACE 20 | 33 |

## Claims

1. A dialkyl peroxide represented by the formula: 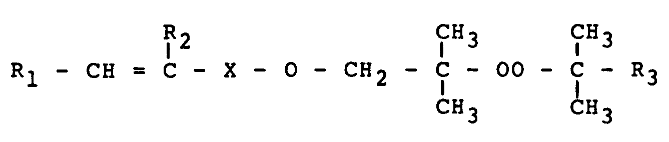 wherein R₁ and R₂ independently stand for a hydrogen atom or a methyl group, R₃ stands for an alkyl group of 1 to 5 carbon atoms, X stands for an alkylene group of 1 to 3 carbon atoms, -C(O)-, CH₂-(O-CH₂-CHR₄)ₘ-OC(O)- [wherein R₄ stands for a hydrogen atom or a methyl group and m stands for an integer in the range of 0 to 10], or -C(O)-(O-CH₂-CHR₅)ₙ-OC(O)- (wherein R₅ stands for a hydrogen atom or a methyl group and n stands for an integer in the range of 1 to 10).

2. A dialkyl peroxide according to claim 1, wherein said dialkyl peroxide is selected from the group consisting of 2-t-butylperoxy-2-methylpropyl methacryloyloxy ethyl carbonate, 2-(1,1-dimethylpropylperoxy)-2-methylpropyl methacryloyloxy ethyl carbonate, 2-(1,1-dimethylbutylperoxy)-2-methylpropyl methacryloyloxy ethyl carbonate, 2-(1,1,3,3,-tetramethylbutylperoxy)-2-methylpropyl methacryloyloxy ethyl carbonate, 2-t-butylperoxy-2-methylpropyl acryloyloxy ethyl carbonate, 2-t-butylperoxy-2-methylpropyl methacryloyloxy isopropyl carbonate, 2-t-butylperoxy-2-methylpropyl methacryloylpoly(oxy isopropyl) carbonate, 2-t-butylperoxy-2-methylpropyl methacryloyloxyethoxy ethyl carbonate, 2-t-butylperoxy-2-methylpropyl methacrylate, 2-t-butylperoxy-2-methylpropyl acrylate, 2-t-butylperoxy-2-methylpropyl methallyl carbonate, 2-t-butylperoxy-2-methylpropyl allyl carbonate, 2-t-butylperoxy-2-methylpropyl crotonate, and 2-t-butylperoxy-2-methyl-propyl allyl ether.

3. A composition for the production of a cross-linked ethylene type polymer, characterized in that said composition has as essential components thereof an ethylene type polymer and a dialkyl peroxide represented by the formula: 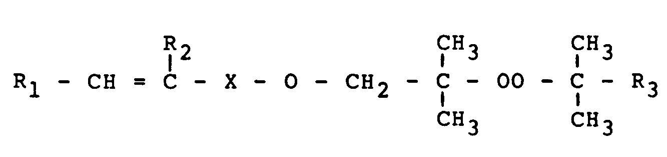 wherein R₁ and R₂ independently stand for a hydrogen atom or a methyl group, R₃ stands for an alkyl group of 1 to 5 carbon atoms, X stands for an alkylene group of 1 to 3 carbon atoms, -C(O)-, CH₂-(O-CH₂-CHR₄)ₘ-OC(O)- [wherein R₄ stands for a hydrogen atom or a methyl group and m stands for an integer in the range of 0 to 10], or -C(O)-(O-CH₂-CHR₅)ₙ-OC(O)- (wherein R₅ stands for a hydrogen atom or a methyl group and n stands for an integer in the range of 1 to 10).

4. A composition according to claim 3, wherein said ethylene type polymer is selected from the group consisting of low-density polyethylene, high-density polyethylene, ethylene-propylene copolymer (EPR), ethylene-butene copolymer, ethylene-pentene copolymer, ethylene-vinyl acetate copolymer (EVA), ethylene-propylene-diene copolymer (EPDM), chlorinated polyethylene, ethylene-ethyl acrylate copolymer (EEA), ethylene-methyl methacrylate copolymer, ethylene-glycidyl methacrylate copolymer (EGMA), and ethylene-acrylonitrile copolymer.

5. A composition for the production of a cross-linked ethylene type polymer according to claim 3, comprising 100 parts by weight of ethylene type polymer and 0.5 to 5 parts by weight of a dialkyl peroxide.

6. A method for cross-linking an ethylene type polymer in the presence of a cross-linking agent, which method is characterized in that said cross-linking agent is a dialkyl peroxide represented by the formula: 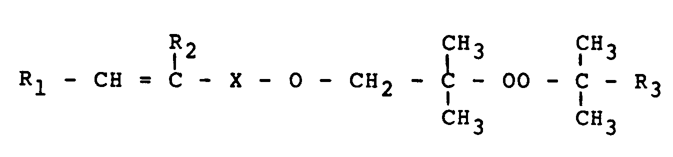 wherein R₁ and R₂ independently stand for a hydrogen atom or a methyl group, R₃ stands for an alkyl group of 1 to 5 carbon atoms, X stands for an alkylene group of 1 to 3 carbon atoms, -C(O)-, -CH₂-(O-CH₂-CHR₄)ₘ-OC(O)- [wherein R₄ stands for a hydrogen atom or a methyl group and m stands for an integer in the range of 0 to 10], or -C(O)-(O-CH₂-CHR₅)ₙ-OC(O)- (wherein R₅ stands for a hydrogen atom or a methyl group and n stands for an integer in the range of 1 to 10).

7. A method according to claim 6, wherein said cross-linking agent is used in an amount in the range of 0.5 to 5 parts by weight, based on 100 parts by weight of said ethylene type polymer.

8. An electric power cable comprising a conductor and at least one resin layer coating said conductor, characterized in that said at least one resin layer is produced by cross-linking an ethylene type polymer with a dialkyl peroxide represented by the formula: 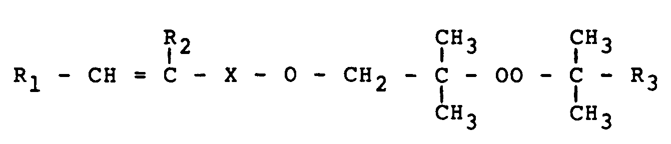 wherein R₁ and R₂ independently stand for a hydrogen atom or a methyl group, R₃ stands for an alkyl group of 1 to 5 carbon atoms, X stands for an alkylene group of 1 to 3 carbon atoms, -C(O)-, -CH₂-(O-CH₂-CHR₄)ₘ-OC(O)- [wherein R₄ stands for a hydrogen atom or a methyl group and m stands for an integer in the range of 0 to 10], or -C(O)-(O-CH₂-CHR₅)ₙ-OC(O)- (wherein R₅ stands for a hydrogen atom or a methyl group and n stands for an integer in the range of 1 to 10), used as a cross-linking agent.

9. An electric power cable according to claim 8, wherein said dialkyl peroxide is contained in an amount in the range of 0.5 to 5 parts by weight, based on 100 parts by weight of said ethylene type polymer.

## Patentansprüche

1. Dialkylperoxid dargestellt durch die Formel 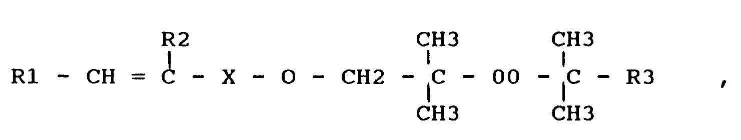 wobei R1 und R2 jeweils für ein Wasserstoffatom oder eine Methylgruppe stehen, R3 für eine Alkylgruppe von 1 bis 5 Kohlenstoffatomen steht, X für eine Alkylengruppe von 1 bis 3 Kohlenstoffatomen, -C(O)-, -CH2-(O-CH2-CHR4)m-OC(O)- [wobei R4 für ein Wasserstoffatom oder eine Methylgruppe und m für eine ganze Zahl zwischen 0 und 10 steht] oder -C(O)-(O-CH2-CHR5)n-OC(O)- (wobei R5 für ein Wasserstoffatom oder eine Methylgruppe und n für eine ganze Zahl zwischen 0 und 10 steht) steht.

2. Dialkylperoxid gemäß Anspruch 1, wobei genanntes Dialkylperoxid aus der folgenden Gruppe ausgewählt wird: 2-t-Butylperoxy-2-methylpropylmethacryloyloxyethyl-carbonat, 2-(1,1-Dimethylpropylperoxy)-2-methylpropyl-methacryloyloxy-ethyl-carbonat, 2-(1,1-Dimethylbutylperoxy)-2-methylpropyl-methacryloyloxyethyl-carbonat, 2-(1,1,3,3,-Tetramethylbutylperoxy)-2-methylpropyl-methacryloyloxy-ethyl-carbonat, 2-t-Butylperoxy-2-methylpropyl-acryloyloxy-ethyl-carbonat, 2-t-Butylperoxy-2-methylpropyl-methacryloyloxyisopropyl-carbonat, 2-t-Butylperoxy-2-methylpropylmethacryloylpoly(oxy-isopropyl)-carbonat, 2-t-Butylperoxy-2-methylpropyl-methacryloyloxyethoxy ethyl-carbonat, 2-t-Butylperoxy-2-methylpropylmethacrylat, 2-t-Butylperoxy-2-methylpropyl-acrylat, 2-t-Butylperoxy-2-methylpropyl-methallyl-carbonat, 2-t-Butylperoxy-2-methylpropyl-allyl-carbonat, 2-t-Butylperoxy-2-methylpropyl-crotonat und 2-t-Butylperoxy-2-methyl-propyl-allyl-ether.

3. Verbindung für die Herstellung eines vernetzten Ethylentyp-Polymerisats, dadurch gekennzeichnet, daß die genannte Verbindung als wesentliche Bestandteile ein Ethylentyp-Polymerisat und ein Dialkylperoxid enthält, dargestellt durch die Formel 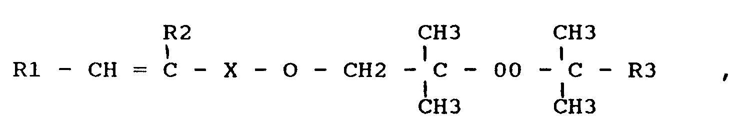 wobei R1 und R2 jeweils für ein Wasserstoffatom oder eine Methylgruppe stehen, R3 für eine Alkylgruppe von 1 bis 5 Kohlenstoffatomen steht, X für eine Alkylengruppe von 1 bis 3 Kohlenstoffatomen, -C(O)-, -CH2-(O-CH2-CHR4)m-OC(O)- [wobei R4 für ein Wasserstoffatom oder eine Methylgruppe und m für eine ganze Zahl zwischen 0 und 10 steht] oder -C(O)-(O-CH2-CHR5)n-OC(O)- (wobei R5 für ein Wasserstoffatom oder eine Methylgruppe und n für eine ganze Zahl zwischen 0 und 10 steht) steht.

4. Verbindung gemäß Anspruch 3, wobei das genannte Ethylentyp-Polymerisat aus der folgenden Gruppe ausgewählt wird: niedrigdichtes (low density) Polyethylen, hochdichtes (high density) Polyethylen, Ethylen-Propylen-Copolymerisat (EPR), Ethylen-Buten-Copolymerisat, Ethylen-Penten-Copolymerisat, Ethylen-Vinylacetat-Copolymerisat (EVA), Ethylen-Propylen-Dien-Copolymerisat (EPDM), chloriniertes Polyethylen, Ethylen-Ethylacrylat-Copolymerisat (EEA), Ethylen-Methylmethacrylat-Copolymerisat, Ethylen-Glycidylmethacrylate-Copolymerisat (EGMA) und Ethylen-Acrylonitril-Copolymerisat.

5. Verbindung für die Herstellung eines vernetzten Ethylentyp-Polymerisats gemäß Anspruch 3, umfassend 100 Gewichtsteile des Ethylentyp-Polymerisats und 0,5 bis 5 Gewichtsteile eines Dialkylperoxids.

6. Verfahren zum Vernetzen eines Ethylentyp-Polymerisats in Gegenwart eines Vernetzungsmittels, wobei das Verfahren dadurch gekennzeichnet ist, daß das Vernetzungsmittel ein Dialkylperoxid ist, dargestellt durch die folgende Formel: 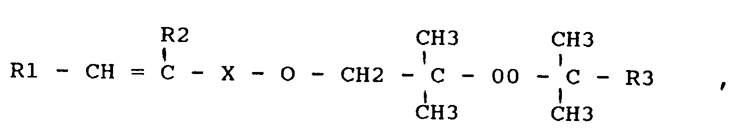 wobei R1 und R2 jeweils für ein Wasserstoffatom oder eine Methylgruppe stehen, R3 für eine Alkylgruppe von 1 bis 5 Kohlenstoffatomen steht, X für eine Alkylengruppe von 1 bis 3 Kohlenstoffatomen, -C(O)-, -CH2-(O-CH2-CHR4)m-OC(O)- [wobei R4 für ein Wasserstoffatom oder eine Methylgruppe und m für eine ganze Zahl zwischen 0 und 10 steht] oder -C(O)-(O-CH2-CHR5)n-OC(O)- (wobei R5 für ein Wasserstoffatom oder eine Methylgruppe und n für eine ganze Zahl zwischen 0 und 10 steht) steht.

7. Verfahren gemäß Anspruch 6, wobei das genannte Vernetzungsmittel in einer Menge im Bereich von 0,5 bis 5 Gewichtsteilen, basierend auf 100 Gewichtsteilen des genannten Ethylentyp-Polymerisats, verwendet wird.

8. Stromkabel, umfassend einen Leiter und mindestens eine den genannten Leiter ummantelnde Harzschicht, dadurch gekennzeichnet, daß genannte mindestens eine Harzschicht durch Vernetzen eines Ethylentyp-Polymerisats mit einem Dialkylperoxid hergestellt wird, dargestellt durch die Formel 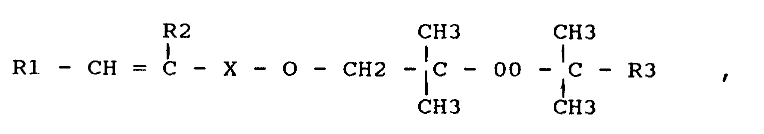 wobei R1 und R2 jeweils für ein Wasserstoffatom oder eine Methylgruppe stehen, R3 für eine Alkylgruppe von 1 bis 5 Kohlenstoffatomen steht, X für eine Alkylengruppe von 1 bis 3 Kohlenstoffatomen, -C(O)-, -CH2-(O-CH2-CHR4)m-OC(O)- [wobei R4 für ein Wasserstoffatom oder eine Methylgruppe und m für eine ganze Zahl zwischen 0 und 10 steht] oder -C(O)-(O-CH2-CHR5)n-OC(O)- (wobei R5 für ein Wasserstoffatom oder eine Methylgruppe und n für eine ganze Zahl zwischen 0 und 10 steht) steht.

9. Stromkabel gemäß Anspruch 8, wobei das genannte Dialkylperoxid in einer Menge im Bereich von 0,5 bis 5 Gewichtsteilen, basierend auf 100 Gewichtsteilen des genannten Ethylentyp-Polymerisats, enthalten ist.

## Revendications

1. Peroxyde de dialcoyle représenté par la formule 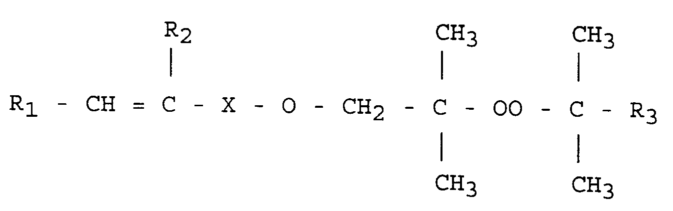 dans laquelle R₁ et R₂ représentent indépendamment un atome d'hydrogène ou un radical méthyle, R₃ représente un radical alcoyle ayant de 1 à 5 atomes de carbone, X représente un groupe alcoylène ayant de 1 à 3 atomes de carbone, -C(O)-, -CH₂-(O-CH₂-CHR₄)ₘ-OC(O)- [dans laquelle R₄ représente un atome d'hydrogène ou un radical méthyle et m représente un nombre entier compris entre 0 et 10] ou -C(O)-(O-CH₂-CHR₅)ₙ-OC(O)- (dans laquelle R₅ représente un atome d'hydrogène ou un radical méthyle et n représente un nombre entier compris entre 0 et 10).

2. Dialcoylperoxyde selon la revendication 1, dans lequel ledit dialcoyl peroxyde est choisi dans le groupe qui consiste en un carbonate de 2-t-butylperoxy 2-méthylpropylméthacryloyloxy éthyle, le carbonate de 2-(1,1-dimethylpropylperoxy)-2- méthyl propyl métha cryloyloxy éthyle, le carbonate de 2-(1,1-diméthylbutyl peroxy)-2- méthylpropyl méthacryloyloxy éthyle, le carbonate de 2-(1,1,3,3-tétra méthylbutylperoxy)-2-méthylpropyl méthacryloyloxy éthyle, le carbonate de 2-t-butylperoxy-2-methylpropyl acryloyloxy éthyle, le carbonate de 2-t-butylperoxy-2 méthylpropylmétha cryloyloxy isopropyle, le carbonate de 2-t-butylperoxy-2-méthylpropyl méthacryloylpoly (oxyisopropyle), le carbonate de 2-t-butylperoxy-2-méthylpropyl méthacryloyloxyethoxy éthyle, le méthacrylate de 2-t-butylperoxy-2-méthylpropyle, l'acrylate de 2-t-butylperoxy 2-méthylpropyle, le carbonate de 2-t-butylperoxy-2-méthylpropyl méthallyl, le carbonate de 2-t-butylperoxy-2-méthylpropyl allyle, le crotonate de 2-t-butylperoxy-2-méthylpropyle, et l'éther de 2-t-butylperoxy-2-méthylpropyle et d'allyl.

3. Composition pour la production d'un polymère du type éthylène réticulé caractérisée en ce que ladite composition a comme composants essentiels de celle-ci, un polymère du type éthylène et un péroxyde de dialcoyle représenté par la formule 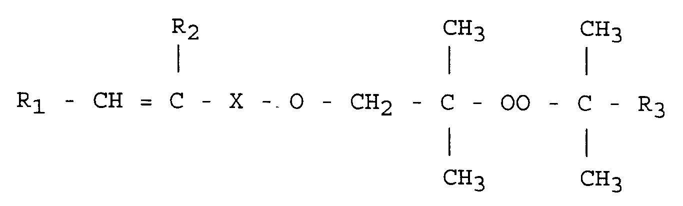 dans laquelle R₁ et R₂ indépendamment représentent un atome d'hydrogène ou un radical méthyle, R₃ représente un radical alcoyle ayant de 1 à 5 atomes de carbone, X représente un groupe alcoylène ayant de 1 à 3 atomes de carbone, -C(O)-, CH₂-(O-CH₂-CHR₄)ₘ-OC(O)- [dans laquelle R₄ représente un atome d'hydrogène ou un radical méthyle et m représente un nombre entier compris entre 0 et 10], ou -C(O)-(OCH₂-CHR₅)ₙ-OC(O) (dans laquelle R₅ représente un atome d'hydrogène ou un radical méthyle et n représente un nombre entier compris entre 1 et 10).

4. Composition selon la revendication 3, dans laquelle ledit polymère du type éthylène est choisi dans le groupe qui consiste en le polyéthylène basse densité, le polyéthylène haute densité, le copolymère éthylène-propylène (EPR), le copolymère éthylène-butène, le copolymère éthylène-pentène, le copolymère éthylène-acétate de vinyle (EVA), le copolymère éthylène propylène-diène (EPDM), le polyéthylène chloré, le copolymère éthylène-acrylate d'éthyle (EEA), le copolymère éthylène-méthacrylate de méthyle, le copolymère éthylène-méthacrylate de glycidyle (EGMA) et le copolymère éthylène-acrylonitrile.

5. Composition pour la production d'un polymère du type éthylène réticulé selon la revendication 3, qui comprend 100 parties en poids de polymère du type éthylène et de 0,5 à 5 parties en poids d'un dialcoylperoxyde.

6. Procédé de réticulation d'un polymère du type éthylène en présence d'un agent de réticulation, ce procédé étant caractérisé en ce que ledit agent de réticulation est un peroxyde de dialcoyle représenté par la formule 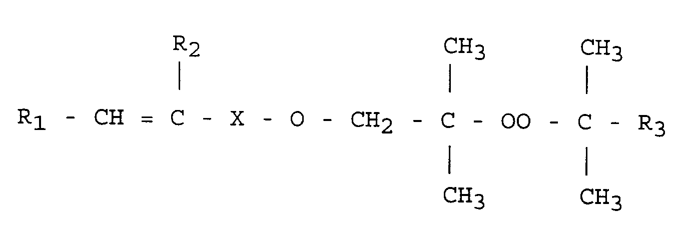 dans laquelle R₁ et R₂, indépendamment, représentent un atome d'hydrogène ou un radical méthyle, R₃ représente un radical alcoyle ayant de 1 à 5 atomes de carbone, X représente un groupe alcoylène ayant de 1 à 3 atomes de carbone, -C(O)-, -CH₂-(OCH₂-CHR₄)ₘ-OC(O)- [dans laquelle R₄ représente un atome d'hydrogène ou un radical méthyle, et m représente un nombre entier compris entre 0 et 10] ou -C(O)-(OCH₂-CHR₅)ₙ-OC(O) (dans laquelle R₅ représente un atome d'hydrogène ou un radical méthyle et n représente un nombre entier compris entre 1 et 10).

7. Procédé selon la revendication 6, dans lequel ledit agent de réticulation est employé dans une quantité allant de 0,5 à 5 parties en poids, basé sur 100 parties en poids dudit polymère du type éthylène.

8. Cable de puissance électrique qui comprend un conducteur et au moins une couche de résine recouvrant ledit conducteur, caractérisé en ce qu'au moins ladite couche de résine est produite en réticulant un polymère du type éthylène avec un peroxyde de dialcoyle représenté par la formule 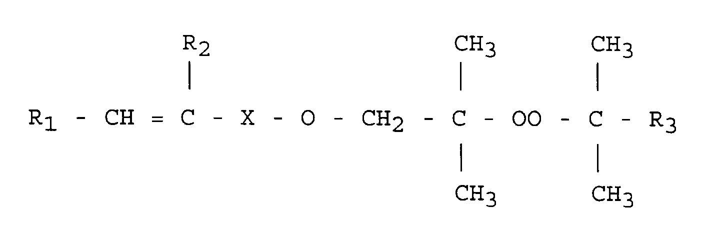 dans laquelle R₁ et R₂ indépendamment représentent un atome d'hydrogène ou un radical méthyle, R₃ représente un raical alcoyle ayant de 1 à 5 atomes de carbone, X représente un radical alcoylène ayant de 1 à 3 atomes de carbone, -C(O)-, -CH₂-(O-CH₂-CHR₄)ₘ-OC(O)- (dans lequel R₄ représente un atome d'hydrogène ou un radical méthyle et m représente un nombre entier compris entre 0 et 10), ou -C(O)-(O-CH₂-CHR₅)ₙ - OC(O)- (dans laquelle R₅ représente un atome d'hydrogène ou un radical méthyle et n représente un nombre entier compris entre 1 et 10, employé en tant qu'agent de réticulation.

9. Câble de puissance électrique selon la revendication 8, dans lequel le peroxyde de dialcoyle est dans une quantité de 0,5 à 5 parties en poids, basé sur 100 parties en poids dudit polymère du type éthylène.
